# EUROPEAN PATENT APPLICATION

(11) **EP 1 867 323 A1**
(43) Date of publication of application: **19.12.2007**
(21) Application number: 06012080.5
(22) Date of filing: 13.06.2006
(51) Int. Cl.: A61K 9/113

(54) **Pharmaceutical compositions with biological barriers permeation enhancing properties**

(71) Applicant: Farmatron Ltd., London W1W 7BL (GB)
(72) Inventor: Pedrani, Massimo, London W1 W7 BL (GB); Carli, Fabio, 34136 Trieste (IT)
(74) Representative: Minoja, Fabrizio

(57) **Abstract**

Pharmaceutical compositions comprising a water/oil/water (W1/O2/W3) or an oil/water/oil (O1/W2/O3) double microemulsion, with droplets size below one micron, with the drug included in the internal water phase W1 or internal oil phase O1, whereas the external oil O2 and second water phase W3 or the external water phase W2 and second oil phase 03 contain substances able to inhibit the enzymes, present in the mucosa or biological barrier to be permeated or physiological environment of administration, capable to degrade the drug or to cause its efflux from the barrier; alternatively the external oil and second water phase or external water phase and second oil phase contain permeation enhancing agents, i.e., substances able to increase the diffusion of the drug through the biological barrier.

## Description

### BACKGROUND OF THE INVENTION

Microemulsions have been recently thoroughly studied as carriers of medicaments. Pharmaceutical compositions based on microemulsions have been proposed for example for the oral, parenteral, trans-mucosal, aerosol and topical administration.

For instance, EP 387647 discloses microemulsions for the transdermal, nasal or rectal drug delivery particularly of dihydroergotamine mesylate.

Parenteral compositions of diclofenac in microemulsion form are disclosed in WO 95/03121.

Water-oil-water microemulsions of antigenic peptides are disclosed in US 6117432.

The use of tocopherols as emulsion vehicle for poorly soluble drugs is disclosed in WO 00/71163.

JP 10330287 disclose a water in oil in water emulsion wherein the inner phase comprises insulin and gelatins whereas the oil phases comprise lecithins, tocopherols, glycerides, a surfactant and docosahexaenoic acid. The disclosed composition, which is not in form of microemulsion, addresses the problem of insulin absorption through the colon and rectum.

WO 99/27918 discloses water/oil/water microemulsions for delivering drugs through the brain blood barrier.

WO 03/013421 and WO 03/51334 disclose double microemulsions incorporated onto a solid support particularly suited for the oral administration in form of capsules and tablets.

None of the prior art documents however addresses neither the problem of the metabolic degradation of the drug at the site of action by inhibiting enzymes or that of the usually low permeation of the drug through the membranes and mucosae. A further factor negatively affecting the absorption of a given drug through tissues is due to the so called drug-efflux phenomenon, determined by specific enzymes located in some mucosae and which actively oppose the absorption of the drug through cells and tissues.

### DESCRIPTION OF THE INVENTION

It has now been surprisingly found that the prior art drawbacks cab be effectively overcome by including into the external phases of double microemulsions suitable enzymes and/or permeation enhancers.

The invention provides therefore water/oil/water or oil/water/oil double microemulsions as carriers of drugs having different solubility characteristics, said microemulsions being characterized in that they comprise agents improving the bioavailability of the drug, either by inhibiting enzymes responsible for the metabolic degradation of the drug at the site of action, by enhancing the permeation of the drug through the membranes and mucosae and/or by inhibiting enzymes responsible for the drug-efflux phenomenon.

The invention also provides pharmaceutical compositions comprising said double microemulsions as carriers of medicaments to be administered oral, topical, transdermal, nasal, pulmonary, transmucosal, vaginal, ocular, rectal application.

Both scarcely water-soluble / hydrophobic drugs and highly polar, water-soluble drugs may be advantageously formulated according to the invention.

### DETAILED DESCRIPTION OF THE INVENTION

The present invention provides pharmaceutical compositions comprising oil/water/oil (O1/W2/O3) or a water/oil/water (W1/O2/W3) double microemulsions wherein the external phases (W2 and 03 for O1/W2/O3, O2 and W3 for W1/O2/W3) contain mucosal/physiological environment enzymes-inhibitors and/or P-gp inhibitors and/or absorption permeation enhancers.

Examples of permeation enhancers contained in the external phases O2 and W3 of W1/O2/W3 double microemulsion or to external phases W2 and O3 of O1/W2/O3 double microemulsion include non-ionic surfactants such as polyoxyalkylene fatty ethers (BRIJ®), (TWEEN® 20, TWEEN® 80, etc.); ionic surfactants such as sodium dodecylsulfate; bile salts (e.g., sodium glycholate, sodium taurocholate, sodium deoxycholate,etc.); fatty acids like oleic acid, palmitoleic acid, linoleic acid, sodium oleate, sorbitan trioleate, polyoxyethylene sorbitan trioleate, etc; sodium salicylate, sodium caprate, diethylmaleate, laurylmaltopyranoside, etc.

Examples of metabolic degradation enzymes inhibitors contained in the external phase O2 and W3 of W1/02/W3 double microemulsion or to the external phases W2 and O3 of O1/W2/O3 double microemulsion are CYP3A inhibitors, protease inhibitors like aprotinin, chymostatin, bacitracin, benzamidine, phosphoramidon, leupeptin, bestatin, leupeptin, cystatin, amastatin, pepstatin, potato carboxypeptidase, soybean trypsin inhibitor, diisopropylfluorophosphate, EDTA.

Examples of drug-efflux P-glycoprotein enzymes inhibitors contained in the external phase O2 and W3 of W1/O2/W3 double microemulsion or in the external phases of W2 and O3 of O1/W2/03 double microemulsion are flavonoids contained in fruit juices such as Naringenin, Isoquercetin, Quercetin, Vitamin E TPGS (Tocopheryl Glycolsuccinate).

The microemulsions of the invention may contain both a permeation enhancing agent and an enzyme inhibitor.

Preferred drugs which may be advantageously formulated in the double microemulsion O1/W2/O3 of the invention are scarcely water-soluble/hydrophobic drugs, with very low polarity and consequent poor solubility in the biological aqueous fluids such as gastro-intestinal content, pulmonary fluids or buccal fluids.

Examples of these drugs are anesthetics, anti-asthma agents, antidepressants, anti-diabetics, anti-epileptics, anti-fungals, anti-gout, anti-neoplastics, anti-obesity agents, anti-protozoals, anti-virals, anti-psychotics, calcium regulating agents, cardiovascular agents, corticosteroids, diuretics, dopaminergic agents, gastrointestinal agents, hormones (peptide and non-peptide), immunosuppressants, lipid regulating agents, phytoestrogens, prostaglandins, relaxants and stimulants, vitamins/nutritionals and xanthines.

Said therapeutic categories include well known compounds such as paclitaxel, docetaxel, etoposide, teniposide, fludarabine, doxorubicin, daunomycin, mitoxanthrone, emodin, 5-fluorouracil, camptothecin, retinoic acids, ubidecarenone, verapamil, cyclosporine, tacrolimus, statins such as lovastatin, atorvastatin, simvastatin, piroxicam, nimesulide, naproxen, ibuprofen, indomethacin, phenytoin, fentanyl, desmopressin, angiotensin I, II and III, enkephalins and their analogs, ACTH, antiinflammatory peptides I, II, III, bradykinin, calcitonin, Interferon, cholecystikinin (CCK) fragments, luteinizing hormone releasing hormone (LHRH), neurokinins (e. g. neurokinin A), somatostatin, substance P, thyroid releasing hormone (TRH), vasopressin, fibrinogen receptor antagonists growth hormone releasing peptides (GHRP), insulin, LH-RH releasers and inhibitors, immunosuppressive anti-TNF alpha-monoclonal antibodies (e.g.Rituximab, Trastuzumab, Infliximab, Gemtuzumab, Alemtuzumab, Ibritumomab, Tositumomab- Iodine 131, Cetuximab, Bevacizumab) endothelins, atrial natriuretic factor, gastrin, MSH modulators, cytokines, renin inhibitors, HIV protease inhibitors, fluconazole, itraconazole, nifedipine, carbamazepine, fluoxetine, griseofulvin, raloxifene, paroxetine, glimepiride, anagrelide, modafinil, losartan, valsartan, cabergoline, replaginide, glipizide, benzodiazepines, clofibrate, chlorpheniramine, digoxin, digitoxin, ergotamine tartate, estradiol, fenofibrate, hydrochlorothiazide, hydrocortisone, medrogeston, oxyphenbutazone, prednisolone, prednisone, polythiazide, progesterone, spironolactone, tolbutamide, 10,11-dihydro-5H-dibenzo[a,d]cyclo-heptene-5-carboxamide, 5H-dibenzo[a,d]cycloheptene-5-carboxamide. In the case of the double microemulsion W1/O2/W3, the preferred drugs are very water-soluble, highly polar, in some case with ionic charges and/or high molecular weight, with consequent poor permeability of biological barriers such as gastro-intestinal or pulmonary or buccal mucosa.

Examples of these drugs are antibiotics, polypeptides, proteins (insulin, erythropoietin, CSF), polynucleotides, acellular vaccines, bisphosphonates (alendronate, ibandronate, clodronate, zoledronate, pamidronate, risedronate, etidronate etc.), enalapril, acyclovir, enfuvirtide, polyphenols, bioflavones, hydrosoluble vitamins, choline, carnitine, carnosine and related peptides, platinum complexes, glycosamineglycans such as heparins or fraction thereof, hyaluronic acid, dermatans, glucosamine.

The oil used for the internal/external phases of the double microemulsions of the invention can be natural, synthetic or semisynthetic. Examples of natural oils include of olive, sunflower, safflower, peanut, corn, soybean, maize, coconut, sesame oils.

Examples of synthetic or semisynthetic oils are esters of short, medium or long chain fatty acids as isopropyl miristate, isopropylpalmitate, ethyl laurate, isopropyl caprilate, isopropyl caprinate, isopropyl laurate, isopropyl stearate, ethyl oleate, oleyl oleate or long chain alcohols or polyols such as hexadecylic alcohol, oleic alcohol, lauric alcohol, cetyl stearylic alcohol, benzyl alcohol, decanoic acid, butanoic acid, silicon oils, mono-, di-and tri-glycerides mixtures or poly-ethoxylated derivatives thereof, polyhydroxyethyl triglycerides, polyhydroxy triglycerides, capricocaprilic triglycerides.

The surfactants can be of natural or synthetic origin; examples of surfactants include sorbitan laurate, sorbitan palmitate, sorbitan stearate (Span 20®, Span 40^{®}, Span 60^{®} respectively), polysorbates such as polyoxyethylene (20) sorbitan monostearate, polyoxyethylene (20) sorbitan monopalmitate, polyoxyethylene (20) sorbitan monolaurate, polyoxyethylene (20) sorbitan monooleate (Tween 60^{®}, Tween 40^{®}, Tween 20^{®}, Tween 80^{®}), polyoxyethylene ethers of fatty acids, e.g. polyoxyethylene 2 cetyl ether, polyoxyethylene 20 cetyl ether (Brij 52^{®}, Brij 58^{®}) and polyoxyethylene hydrogenated castor oil 40 (HCO-40^{®}). Polyvinyl alcohol may also be used as amphiphylic surfactant.

Combinations of surfactants with different chemical characteristics can also be used.

The cosurfactants can either be synthetic or natural. Examples of synthetic cosurfactants include short chain alcohols such as ethanol, isopropanol, n-butanol, ethanediol, 1,2-propanediol, 1,3-propanediol, 1,3-butane diol, 1,4-butane diol, glycerine, polyethylene glycols (e.g. PEG 400, PEG 600) butyric acid, valerianic acid, capronic acid, benzyl acid, decanoic acid, lauric acid, lauryl alcohol.

Examples of natural cosurfactants include lecithins and phospholipids.

The external water (w/o/w) phase of the double microemulsions of the invention (W1/O/W3) consists of water as such or buffered at different pH's and ionic strengths, of mixtures of water and polyethylenglycols, polyol glycerides, propylene glycol, tetra glycol, ethoxy glycol, mixtures of water and polyvinylpyrrolidone, polyacrylic acids and derivatives, polymethacrylic acids and derivatives, alginic acids and derivatives, polyvinylalcohol, chitosan and derivatives, xanthan and derivatives, guar gum, arabic gum, dextran, cellulose and derivatives, starch and derivatives.

The internal aqueous phase of the double microemulsions of the invention (W2 for O1/W2/O3) consists of water as such or buffered at different pH's and ionic strengths or of mixtures of water and polyethyleneglycols, polyglycol glycerides, propylene glycol, tetra glycol, ethoxy glycol.

The double microemulsions (O1/W2/O3 or W1/O2/W3) of the invention have the following preferred compositions by weight percentage:
a) double microemulsion O1/W2/O3:
   a1) oil (O1, internal phase) from 1.0% to 5.0%.
   a2) water or aqueous solution (W2) from 3.0% to 10%.
   a3) oil (03, external phase) from 60% to 90%
   a4) metabolic enzymes-inhibitor, P-gp-inhibitor, absorption enhancer from 0.05% to 10.0%.
   a5) drug from 0.01% to 15%.
   a6) surfactant from 2.0% to 20%.
   a7) cosurfactant from 0% to 5.0%.
b) double microemulsion W1/O2/W3 :
   b1) water or aqueous solution (W1, internal phase) from 1.0% to 5.0%.
   b2) oil (02 external phase) from 6.0% to 15%.
   b3) water or aqueous solution (W3, external phase) from 60% to 90%.
   b4) metabolic enzymes inhibitor, P-gp-inhibitor, absorption enhancer from 0.05% to 10.0%.
   b5) drug from 0.01% to 15%.
   b6) surfactant from 2.0% to 20%.
   b7) cosurfactant from 0% to 5.0%.

The compositions of the invention can be prepared by a process comprising:
a. solubilization of the mucosal/physiological environment enzymes-inhibitor, P-gp-inhibitor, absorption permeation enhancer in the external phase/phases of the double microemulsion of choice (W2 and 03 for O1/W2/O3, O2 and W3 for W1/O2/W3);
b. solubilization of the drug in the internal phase of the double microemulsion of choice (O1 of the O1/W2/O3 in the case of hydrophobic, scarcely water soluble drug; W1 of the W1/O2/W3 in the case of water soluble, poorly permeable drugs);
c. addition of the oil or water drug solution of stage b) to the water or oil solution of stage a);
d. addition of surfactant/cosurfactant to the mixture of stage c and agitation with the formation of the O1/W2 or W1/O2 microemulsion;
e. addition of the microemulsion O1/W2 or W1/O2 of stage d) to the oil (03) phase or water (W3) phase containing surfactant/cosurfactant and agitation with the formation of the double microemulsion O1/W2/O3 or W1/O2/W3 with mucosal/physiological environment. enzymes-inhibitors and/or P-gp-inhibitors and/or absorption permeation enhancers in the W2 and O3 of the O1/W2/O3 or O2 and W3 of the W1/O2/W3 double microemulsion.

Possible variations to this process can be introduced in relation to specific experimental conditions.

For example, in some cases, the mucosal/physiological environment enzymes-inhibitor/P-gp-inhibitor/absorption enhancer cannot be completely solubilized in the oil or water external phase of choice: in this case the substance will be dispersed in the suitable external phase.

It is also possible that the drug is dispersed in the internal phase of choice when it is not completely soluble in the internal oil or water phase of choice.

If desired, a viscosity-increasing substance can be added to the second external phase of the double microemulsions (W3 for the W1/O2/W3, 03 for the O1/W2/O3) such as polymers such as hydroxypropylcellulose, chitosan, polyvinylpyrrolidone, polymethylmethacrylate.

Said viscosity-increasing substances can also be added to the other phases of the double microemulsions.

In some cases, these viscosity-increasing polymers may also have bioadhesive properties so to increase residence-time in the biological environment of action (e.g., stomach, eye sac, mouth cavity, etc.) or to guarantee more intimate contact with the biological barrier to be overcome, e.g., gastro-intestinal tract mucosa, pulmonary tissues, etc.

The compositions according to the invention may be formulated with the addition of conventional excipients as dosage forms for oral, topical, transdermal, nasal, pulmonary, transmucosal, vaginal, ocular, rectal application.

The compositions according to the invention may also be formulated in dosage forms after the addition of said compositions onto solid adsorbent particles.

The compositions of the present invention present unexpected improved properties both in terms of biopharmaceutical properties (particle size of droplets in the nanometers range with consequent extremely high surface area, high solubility of hydrophobic drugs in the oil components, higher diffusion tendency through hydrophobic membranes of very polar drugs) and in terms of a much lower metabolic degradation or P-glycoprotein efflux effects with consequent much higher biological membranes/barriers permeation tendency.

The invention is disclosed in more detail in the following some examples.

### EXAMPLE 1

### a) w/o formulation

A w/o microemulsion was prepared by mixing using a paddle mixer at the speed of 250 rpm at 25°C for 1 h an aqueous phase containg erythropoietin dissolved (300 µg/g) to an oily phase Lauroglycol FCC^{®}:Labrasol^{®} (1:1) containing chymostatin (200 µg/g) and the surfactant Tween 20^{®} (1.04 g).

| | |
|---|---|
| Erythropoietin | 0.00061 g |
| Water | 2.03 g |
| Polyetyhlene glycol monoester of lauric acid (Lauroglycol FCC^{®}) | 6.01 g |
| Mixture of mono-,di- and triglycerides and mono- and di-fatty esters of polyethylene glycol (Labrasol^{®}) | 6.01 g |
| Chymostatin | 0.0024 g |
| Polyoxyethylene (20) sorbitan monolaurate (Tween 20^{®}) | 1.04 g |

### b) w/o/w formulation

The microemulsion a) w/o was added to an aqueous phase (75.45g) under paddle stirring at the speed of 300 rpm for 0.75 h, Tween 20^{®} was then added (4.04 g) under paddle stirring at the speed of 280 rpm for 0.5 h to obtain a double microemulsion w/o/w. The composition of the resulting w/o/w double microemulsion was the following:

### Example 1 w/o/w composition

| | |
|---|---|
| Erythropoietin | 0.00061 g |
| Polyetyhlene glycol monoester of lauric acid (Lauroglycol FCC^{®}) | 6.01 g |
| Mixture of mono-di and triglycerides and mono and di-fatty esters of polyethylene glycol (Labrasol^{®}) | 6.01 g |
| Chymostatin | 0.0024 g |
| Polyoxyethylene (20) sorbitan monolaurate (Tween 20^{®}) | 5.08 g |
| Water | 77.48 g |

### EXAMPLE 2

### a) w/o formulation

A w/o microemulsion was prepared by mixing using a paddle mixer at the speed of 240 rpm at 25°C an aqueous phase containg sodium alendronate dissolved (0.5 g/g) with an oily phase containg Akoline^{®} :Labrasol^{®}:TaurumDeoxycholate in ratio 1:1:0.09, and the surfactant Tween 80^{®} (0.653g).

| | |
|---|---|
| Sodium Alendronate | 0.492 g |
| Water | 0.984 g |
| Monoglycerides of caprylic/capric acids (Akoline^{®}). | 1.88 g |
| Mixture of mono-di and triglycerides and mono and di-fatty esters of polyethylene glycol (Labrasol^{®}) | 1.88 g |
| Taurum Deoxycholate | 0.168 g |
| Polyoxyethylene (20) sorbitan monooleate (Tween 80^{®}) | 0.653 g |

### b) w/o/w formulation

The microemulsion a) w/o was added to an aqueous phase (33.39g) under paddle stirring at the speed of 300 rpm for 0.5 h, Tween 80^{®} was then added (2.50 g) under paddle stirrimg at the speed of 280 rpm for 0.75 h to obtain a double microemulsion w/o/w. The composition of the resulting w/o/w double microemulsion was the following:

### Example 2 w/o/w composition

| | |
|---|---|
| Sodium Alendronate | 0.492 g |
| Monoglycerides of caprylic/capric acids (Akoline^{®}) | 1.88 g |
| Mixture of mono-di and triglycerides and mono and di-fatty esters of polyethylene glycol (Labrasol^{®}) | 1.88 g |
| Taurum Deoxycholate | 0.168 g |
| Polyoxyethylene (20) sorbitan monooleate (Tween 80^{®}) | 3.153 g |
| Water | 33.39 g |

### EXAMPLE 3

### a) w/o formulation

A w/o microemulsion was prepared by mixing using a paddle mixer at the speed of 220 rpm at 25C an aqueous phase containg somatostatin dissolved (0.4 g/g), to an oily phase, Labrafac CC^{®}:Egg Lecithin (1:0.005), containing CYP3A inhibitor as hesperidin (100 µg/g), and the surfactant Span 40^{®} (2.04 g).

| | |
|---|---|
| Somatostatin | 0.82 g |
| Water | 2.05 g |
| Hesperidin | 0.0008 g |
| Sorbitan palmtate (Span 40^{®}) | 2.04 g |
| Triglycerides of caprylic/capric acids (Labrafac CC^{®}) | 8.00 g |
| Egg lecithin | 0.04 g |

### b) w/o/w formulation

The microemulsion a) w/o was added to an aqueous phase (72.78 g) under paddle stirring at the speed of 310 rpm for 0.5 h, Span 40^{®} was then added (5.03 g) under paddle stirring at the speed of 280 rpm for 0.5 h to obtain a double microemulsion w/o/w. The composition of the resulting w/o/w double microemulsion was the following:

### Example 3 w/o/w composition

| | |
|---|---|
| Somatostatin | 0.82 g |
| Triglycerides of caprylic/capric acids (Labrafac CC^{®}) | 8.00 g |
| Hesperidin | 0.0008 g |
| Sorbitan palmitate (Span 40^{®}) | 7.07 g |
| Egg Lecithin | 0.04 g |
| Water | 74.83 g |

### EXAMPLE 4

### a) o/w microemulsion formulation

A o/w microemulsion was prepared by mixing using a paddle mixer at the speed of 220 rpm for 0.5 h at 25°C an oily phase (Akoline^{®}:Transcutol HP^{®}7:3) containg simvastatin dissolved (0.34 g/g), to a water phase, the surfactant Tween 80^{®} was subsequently added with a mixing rate of 250 rpm for 0.5 h; the composition of the resulting microemulsion was:

| | |
|---|---|
| Simvastatin | 0.366 g |
| Monoglycerides of caprylic/capric acids(Akoline^{®}) | 0.754 g |
| Diethylene glycol monoethyl ether (Transcutol HP^{®}) | 0.323 g |
| Polyoxyethylene (20) sorbitan monooleate (Tween 80^{®}) | 0.303 g |
| Water | 3.230 g |

### b) o/w/o double microemulsion

The o/w microemulsion a) was added under stirring (300 rpm) for 0.75 h to a an oily phase (Akoline^{®}), the surfactant Tween 80^{®} (3.01 g) was added under paddle stiring at speed of 270 rpm for 0.5 h, obtaining a double microemulsion containing simvastatin.

| | |
|---|---|
| Simvastatin | 0.367 g |
| Monoglycerides of caprylic/capric acids (Akoline^{®}) | 23.804 g |
| Diethylene glycol monoethyl ether (Transcutol HP^{®}) | 0.323 g |
| Polyoxyethylene (20) sorbitan monooleate (Tween 80^{®}) | 3.313 g |
| Water | 3.230 g |

### EXAMPLE 5

### a) o/w microemulsion formulation

A o/w microemulsion was prepared by mixing using a paddle mixer at the speed of 220 rpm for 0.75 h at 25°C an oily phase (Lauroglycol FCC^{®}) containing quercetin (0.05 g/g) and dissolved Itraconazole (0.44 g/g) to a water phase, the surfactant Tween 80^{®} was subsequently added with a mixing rate of 250 rpm for 0.5 h; the composition of the resulting microemulsion was:

| | |
|---|---|
| Itraconazole | 1.35 g |
| PolyethyleneGlycolmonoester of lauric acid (Lauroglycol FCC^{®}) | 3.05 g |
| Quercetin | 0.15 g |
| Polyoxyethylene (20) Sorbitan Monooleate (Tween 80^{®}) | 2.05 g |
| Water | 6.05 g |

### b) o/w/o double microemulsion

The o/w microemulsion a) was added under stirring (300 rpm) for 0.5 h to an oily phase(Lauroglycol^{®}:Quercetin) (55.90), the surfactant Tween 80^{®} (4.01 g) was added under paddle stirring at speed of 270 rpm for 0.75 h, obtaining a double microemulsion containing itraconazole.

| | |
|---|---|
| Itraconazole | 1.35 g |
| PolyethyleneGlycolmonoester of lauric acid (Lauroglycol FCC^{®}) | 53.10 g |
| Quercetin | 2.80 g |
| Polyoxyethylene (20) Sorbitan Monooleate (Tween 80^{®}) | 6.06 g |
| Water | 6.05 g |

### EXAMPLE 6

### a) o/w microemulsion formulation

A o/w microemulsion was prepared by mixing using a paddle mixer at the speed of 280 rpm for 0.50 h at 25°C an oily phase (Plurol oleique CC497^{®}) containing Quercetin (0.02 g/g) and dissolved danazole (0.40 g/g) to a water phase, the surfactant Tween 80^{®} was subsequently added with a mixing rate of 240 rpm for 0.5 h; the composition of the resulting microemulsion was:

| | |
|---|---|
| Danazol | 0.408 g |
| Polyglyceril-6 dioleate (Plurol oleique^{®} CC497) | 1.02 g |
| Quercetin | 0.0204 g |
| Polyoxyethylene (20) sorbitan monooleate (Tween 80^{®}) | 0.700 g |
| Water | 5.022 g |

### b) o/w/o double microemulsion

The o/w microemulsion a) was added under stirring (300 rpm) for 0.5 h to a an oily phase(Plurol oleique CC497^{®}) (26.96 g), the surfactant Tween 80^{®} (2.02 g) was added under paddle stiring at speed of 280 rpm for 0.50 h, obtaining a double microemulsion containing itraconazole

| | |
|---|---|
| Danazol | 0.408 g |
| Polyglyceril-6 dioleate (Plurol oleique^{®} CC497) | 27.980 g |
| Quercetin | 0.0204 g |
| Polyoxyethylene (20) sorbitan monooleate (Tween 80^{®}) | 2.720 g |
| Water | 5.022 g |

### CHARACTERIZATION TESTS

The double microemulsions of the invention were characterized by the following methods.

### - Size determination of the droplets of double microemulsions of the invention

The size of the droplets of the double microemulsions prepared as shown in the examples 1-6 was determined by Laser Light Scattering (Coulter Counter, Mod. N4 Plus); data are reported in Table 1.

**Table 1 - Droplets size of the double microemulsions of the invention**

| **Sample** | **Droplets Size** |
|---|---|
| Example 1 | 1 85 nm |
| Example 2 | 230 nm |
| Example 3 | 120 nm |
| Example 4 | 285 nm |
| Example 5 | 210 nm |
| Example 6 | 6 305 nm |

### - Solubilization kinetics of the double microemulsions of the invention

The "in vitro" solubilization kinetics was determined in buffer solution (pH 7.4, 37°C); sample of the double microemulsion under testing is dispersed in tubes filled with 50 ml of buffer solution and placed over a thermostated shaking plate; at predetermined time intervals samples of the solution were filtered and then ultra-centrifuged; drug concentration was determined by HPLC,data are reported in Table 2.

**Table 2 - Solubilization kinetics of the double microemulsions of the invention**

| **Sample** | **Drug concentration (ug / ml)** | | | | |
|---|---|---|---|---|---|
| | **0.5 hr** | **1 hr** | **3 hr** | **8 hr** | **24 hr** |
| Example 5 (Itraconazole) | 5 | 11 | 16 | 20 | 25 |
| Itraconazole pure dispersion | 0.05 | 0.1 | 0.3 | 0.5 | 1.5 |
| Example 6 (Danazol) | 25 | 58 | 72 | 85 | 94 |
| Danazol pure dispersion | 0.2 | 0.5 | 1.0 | 1.5 | 2.0 |

| | | | | | |
|---|---|---|---|---|---|
| **- "In vivo" permeation test** | | | | | |

The permeation test was carried out on anasthetized Wistar rats; the intestinal tract was isolated and incannulated starting form the Treitz ligament (entrance cannula) to a point at 20-30 cm of distance (exit cannula); the experimental double microemulsion under analysis was dispersed in a pH 7.4, 37°C buffer solution and then perfused; the drug concentration remaining in the perfusion liquid was analyzed by HPLC at predetermined time intervals; apparent permeability (Pa) values are derived from the decrease over time of drug concentration in the perfusion liquid (dC/dt =Pa. Cin-Cfin). Data relative to alendronate in double microemulsion of invention are reported in Table 3.

**Table 3 - Apparent permeability of Alendronate Sodium**

| **Sample** | **Pa, Apparent Permeability (cm/sec)** |
|---|---|
| Alendronate solution | 0.15 x 10-4 |
| Alendronate in double micro-emulsion of Example 1 | 14.5 x 10-4 |

## Claims

1. Pharmaceutical compositions comprising a water/oil/water (W1/O2/W3) or an oil/water/oil (O1/W2/O3) double microemulsion whose internal water phase W1 or internal oil phase O1 contain a drug and whose external oil 02 and second waterW3 phase or external water W2 and second oil 03 phase contain either inhibitors of enzymes present in the biological barrier/environment or permeation enhancing agents.

2. The compositions of claim 1 comprising a water/oil/water (W1/O2/W3) double microemulsion.

3. The compositions of claim 1 comprising a oil/water/oil (O1/W2/O3) double microemulsion.

4. The compositions of claims 1 and 3, **characterised in that** said water internal (w/o) phase consists of water as such or buffered at different pH's and ionic strengths, of mixtures of water and polyethyleneglycols, polyglycol glycerides, propylene glycol, tetra glycol, ethoxy glycol.

5. The compositions of claims 1 and 2, **characterised in that** said second external water (w/o/w) phase consists of water as such or buffered at different pH's and ionic strengths, of mixtures of water and polyethylenglycols, polyol glycerides, propylene glycol, tetra glycol, ethoxy glycol, mixtures of water and polyvinylpyrrolidone, polyacrylic acids and derivatives, polymethacrylic acids and derivatives, alginic acids and derivatives, polyvinylalcohol, chitosan and derivatives, xanthan and derivatives, guar gum, arabic gum, dextran, cellulose and derivatives, starch and derivatives.

6. The compositions according to any one of claims from 1 to 5, **characterised in that** said enzyme inhibitor is selected from the group consisting of inhibitors of metabolic degradation enzymes, protease inhibitors or inhibitors of drug efflux enzymes.

7. The compositions of claim 6, **characterised in that** said permeation enhancer is selected from the group consisting of surfactants, bile salts, fatty acids sodium oleate, polyoxyethylene oleyl ether, sorbitan trioleate, polyoxyethylene sorbitan monooleate, polyoxyethylene sorbitan trioleate. sodium salicylate, sodium caprate, diethylmaleate, laurylmaltopyranoside.

8. The compositionsof claims 6, **characterised in that** said metabolic degradation enzymes inhibitors are selected from CYP3A inhibitors, aprotinin, chymostatin, bacitracin, benzamidine, phosphoramidon, leupeptin, bestatin, amastatin, pepstatin, potato carboxypeptidase inhibitor, soybean trypsin inhibitor, diisopropylfluorophosphate.

9. The compositions of claim 6 **characterised in that** said drug efflux enzymes inhibitors include P-glycoproteins inhibitors selected from Naringenin, Isoquercetin, Quercetin, Vitamin E TPGS (Tocopheryl Glycolsuccinate).

10. The compositions according to any one of claims from 1 to 9, **characterised in that** said permeation enhancing agent and enzyme inhibitor are present in the same formulation.

11. The composition according to claim 2 and any one of claims from 4 to 10 and any one of claims from 4 to 8 having the following composition:
a1) water or aqueous solution (W1, internal phase) from 1.0% to 5.0%.
a2) oil (O2 external phase) from 6.0% to 15%.
a3) water or aqueous solution (W3, external phase) from 60% to 90%.
a4) metabolic enzymes inhibitor, P-gp-inhibitor, absorption enhancer from 0.05% to 10.0%.
a5) drug from 0.01 % to 15 %.
a6) surfactant from 2.0% to 20%.
a7) cosurfactant from 0% to 5.0%.

12. The composition according to claim 3 and any one of claims from 4 to 10 having the following composition:
a1) oil (O1, internal phase) from 1.0% to 5.0%.
a2) water or aqueous solution (W2) from 3.0% to 10%.
a3) oil (O3, external phase) from 60% to 90%
a4) metabolic enzymes-inhibitor, P-gp-inhibitor, absorption enhancer from 0.05% to 10.0%.
a5) drug from 0.01% to 15%.
a6) surfactant from 2.0% to 20%.
a7) cosurfactant from 0% to 5.0%.

13. The compositions according to any one of claims from 1 to 12, **characterised in that** said oil is selected from the group of olive, sunflower, safflower, peanut, corn, soybean, maize, coconut, sesame oils, isopropyl miristate, isopropyl palmitate, isopropyl caprilate, isopropyl caprinate, isopropyl laurate, isopropyl stearate, ethyl oleate, oleyl oleate, hexadecylic alcohol, oleic alcohol, lauric alcohol, cetyl stearylic alcohol, benzyl alcohol, decanoic acid, butanoic acid, silicon oils, mono-, di-and tri-glycerides mixtures or poly-ethoxylated derivatives thereof, polyhydroxyethyl triglycerides, polyhydroxy triglycerides, capricocaprilic triglycerides.

14. The compositions according to any one of claims from 1 to 13, **characterised in that** surfactant is selected from the group consisting of anionic, cationic, non-ionic or amphiphilic surfactants.

15. The compositions according to claim 14 wherein the non-ionic surfactants are selected from polyoxyethylene sorbitan esters, polysorbates, polyoxyethylene fatty acids esters, polyoxyl castor oil derivatives, sorbitan esters.

16. The compositions according to claim 15 wherein the surfactants are selected from sorbitan laurate, sorbitan palmitate, sorbitan stearate (Span 20^{®}, Span 40^{®}, Span 60^{®} respectively), polyoxyethylene (20) sorbitan monostearate, polyoxyethylene (20) sorbitan monopalmitate, polyoxyethylene (20) sorbitan monolaurate, polyoxyethylene (20) sorbitan monooleate (Tween 60®, Tween 40^{®}, Tween 20^{®}, Tween 80^{®}), polyoxyethylene 2 cetyl ether, polyoxyethylene 20 cetyl ether (Brij 52^{®}, Brij 58^{®}) and polyoxyethylene hydrogenated castor oil 40 (HCO-40^{®}).

17. The compositions according to claim 14 wherein the anionic surfactants are selected from sodium dodecyl sulfate and dioctyl sodium sulfosuccinate, polyethyleneglycol-glycerol esters, polyglyceride esters, saccharide and fatty acids esters, polyethyleneglycol derivatives.

18. The compositions according to any one of claims from 1 to 17, further comprising a cosurfactant selected from the group consisting of lecithins, phospholipids, ethanol, isopropanol, butanol, ethanediol, 1,2-propanediol, 1,3-propanediol, 1,3-butane diol, 1,4-butane diol, glycerine, polyethylene glycols, butyric acid, valerianic acids, capronic acids, benzyl acids, decanoic acids, lauric acids, lauryl alcohol.

19. The compositions according to any one of claims from 1 to 18 **characterised in that** said drug is selected from the group of anesthetics, anti-asthma agents, antidepressants, anti-diabetics, anti-epileptics, anti-fungals, anti-gout, anti-neoplastics, anti-obesity agents, anti-protozoals, anti-virals, anti-psychotics, calcium regulating agents, cardiovascular agents, corticosteroids, diuretics, dopaminergic agents, gastrointestinal agents, hormones (peptide and non-peptide), immunosuppressive anti-TNF-alpha monoclonal antibodies, immunosuppressants, lipid regulating agents, phytoestrogens, prostaglandins, relaxants and stimulants, vitamins/nutritionals, xanthines, diphosphonates, glycosaminoglycans, polyphenols, bioflavones, hydrosoluble vitamins, glucosamine, platinum complexes, antibiotics, choline or derivatives, carnosine or related peptides, vaccines.

20. The compositions according to any one of claims from 1 to 19, formulated with the addition of excipients as dosage forms for oral, topical, transdermal, nasal, pulmonary, transmucosal, vaginal, ocular, rectal application.

21. The compositions according to claim 20, formulated in dosage forms after the addition of said compositions onto solid adsorbent particles.
